# EUROPEAN PATENT SPECIFICATION

(11) **EP 4 132 392 B1**
(45) Date of publication and mention of the grant of the patent: **28.08.2024**
(21) Application number: 20727366.5
(22) Date of filing: 08.04.2020
(51) Int. Cl.: A61B 17/70

(54) **INTERSPINOUS VERTEBRAL DISTRACTOR**
INTERSPINALER WIRBELDISTRAKTOR
DISTRACTEUR VERTÉBRAL INTERÉPINEUX

(43) Date of publication of application: 15.02.2023
(60) Divisional application: 23201042.1
(73) Proprietor: Diametros Medical S.r.l., 00138 Roma (IT)
(72) Inventor: GARLATTI, Giovanni, 50026 San Casciano in Val di Pesa (Firenze) (IT); FORTUNA, Lorenzo, 50060 Pelago (Firenze) (IT)
(74) Representative: Lunati & Mazzoni S.r.L.
(86) International application number: PCT/IB2020/053350
(87) International publication number: WO 2021/205209

(56) References cited:
- WO-A1-2012/069877
- US-A1- 2008 108 990
- US-A1- 2009 254 185
- US-A1- 2016 206 352
- US-A1- 2017 348 028

## Description

This invention concerns an interspinous vertebral distractor of the type specified in the preamble of the first claim.

In particular, the invention relates to an implant designed to be implanted, preferably percutaneously, in an interspinous space to space two adjacent vertebrae apart.

As is well known, interspinous distraction can be surgically achieved by introducing an interspinous vertebral distractor between two spinous processes that is controlled by an operator-controlled device for inserting and controlling the distractor.

Distractors are prostheses designed to be implanted in the space between the spinous processes of two adjacent vertebrae, in order to maintain an intervertebral distraction that is suitable for limiting the loads transmitted between the vertebrae in case of intervertebral disc degenerative diseases, thus limiting associated pain.

They mainly consist of a base body that can be arranged between the spinous processes and the distractor's expansion anchoring means. The expansion of the anchoring means is controlled by the control device.

The interspinous vertebral distraction procedure consists in positioning a distractor in the space between the spinous processes of two adjacent vertebrae. In detail, with the patient in the prone position, the affected intervertebral space is identified, the spinous processes are brought closer through expansion cannulae, the measurement of the distractor to be inserted is determined by means of a probe and, finally, the selected distractor is inserted.

A first example of a procedure and distraction device and, in particular, a distractor is described in WO2006102269. In this case the distractor has a first ogive end so as to penetrate the intervertebral space, which is formed by a first pair of wings that open; the opposite end is equipped with a second pair of fixed wings enclosing the column between the two pairs of wings.

Another example is described in document US8998955 wherein there are two pairs of mobile wings. Each wing has one end hinged to the base body and the other end is free. To carry out the locking, this patent prescribes rotating the wings at angles greater than 120° so as to have the free ends facing the vertebrae.

An additional example is described in WO2012069877 where locking and the relative ease of introduction and extraction are achieved by means of hooked wings. The described prior art comprises some significant drawbacks.

In particular, despite the solutions outlined above, the interspinous vertebral distraction procedure/device, and, in particular, the distractor, are relatively complicated and, therefore, particularly expensive. This is mainly due to the fact that during the procedure, the operator encounters particular difficulties in inserting and defining a stable positioning of the interspinous vertebral distractors.

Another drawback is the low stability of the interspinous vertebral distractors once they have been implanted.

An equally important drawback is the fact that the control kinematics of the mobile wings is particularly complex.

Another drawback is, thus, the costs and difficulties in implementing the known interspinous vertebral distractors.

In this context, the technical task underlying this invention is to devise an interspinous vertebral distractor able to substantially overcome at least some of the drawbacks mentioned above.

Said technical task comprises the important purpose of the invention's having an interspinous vertebral distractor that, once implanted, is easy to insert and stable, and that is easy to implement and, therefore, costs less.

The technical task and the specified purposes are achieved by an interspinous vertebral distractor as claimed in Claim 1. Preferred embodiments are described in the dependent claims.

Associated surgical procedures are also described herein to aid understanding the invention. These procedures do not form part of the claimed invention.

The characteristics and advantages of the invention are clarified by the following detailed description of preferred embodiments thereof, with reference to the accompanying drawings, wherein:
**Fig. 1** shows, to scale, an interspinous vertebral distractor according to the invention;
**Figs. 2a-2e** illustrate, to scale, the distractor in a sequence during an interspinous vertebral distraction procedure;
**Fig. 3** shows, to scale, a cross-section of the distractor in Fig. 1;
**Fig. 4** shows, to scale, an assembly of an interspinous vertebral distraction device according to the invention;
**Fig. 5** illustrates, to scale, the assembly in Fig. 4;
**Figs. 6a-6f** represent, to scale, a preparation sequence for the interspinous vertebral distraction device according to the invention; and
**Fig. 7** schematises the interspinous vertebral distraction procedure.

In this document, the measures, values, shapes, and geometric references (such as perpendicularity and parallelism), when used with words like "about" or other similar terms such as "approximately" or "substantially", are to be understood as except for measurement errors or inaccuracies due to production and/or manufacturing errors and, above all, except for a slight divergence from the value, measure, shape, or geometric reference which it is associated with. For example, if associated with a value, such terms preferably indicate a divergence of no more than 10% of the value itself.

Furthermore, when used, terms, such as "first", "second", "higher", "lower", "main", and "secondary" do not necessarily identify an order, relationship priority, or relative position, but they can simply be used to distinguish different components more clearly from one another.

The measurements and data provided in this text are to be considered as performed in ICAO International Standard Atmosphere (ISO 2533), unless otherwise indicated.

With reference to the figures, the reference number **1** indicates, as a whole, the interspinous vertebral distractor according to the invention.

The distractor 1 is a percutaneous device that can be implanted for interspinous distraction. It is designed to be implanted in an interspinous space so that it is interposed between two adjacent vertebrae and, thus, spaces two spinous processes apart.

The distractor 1 defines a longitudinal axis **1a** that is, suitably, barycentric.

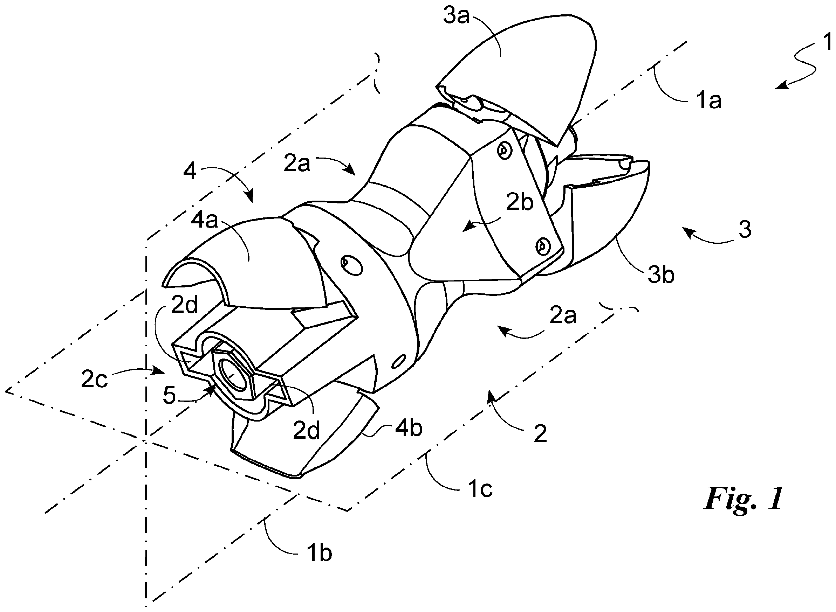

It defines a sagittal plane **1b** that is, suitably, barycentric; and/or a frontal plane **1c** that is, suitably, barycentric (Fig. 1).

The sagittal plane 1b is perpendicular to the frontal plane 1c.

The intersection between the sagittal plane 1b and the frontal plane 1c defines the axis 1a.

The distractor 1 comprises a base body **2** that is designed to be placed between two spinous processes by spacing them apart.

The base body 2 defines, in relation to the longitudinal axis 1a, a first end and a second end.

The base body 2 has a sagittal section (i.e. defined by the intersection of the sagittal plane 1b with the base body 2) with at least one convexity defining a depression **2a** housing a spinous process.

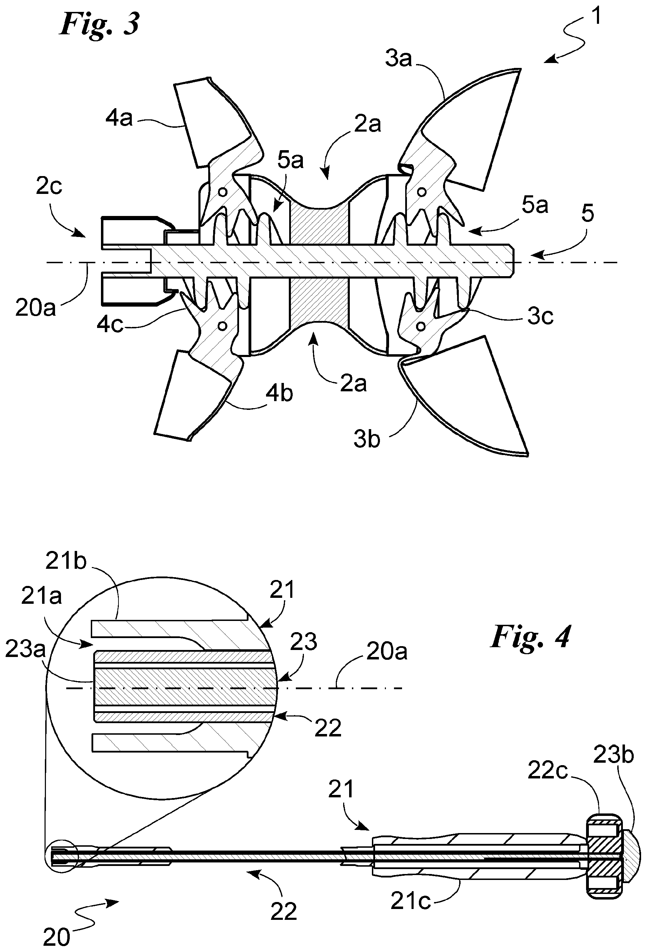

In detail, and as illustrated in Fig. 3, said sagittal section has two convexities and, therefore, two depressions 2a placed opposite the frontal plane 1c and each one is designed to accommodate a spinous process. The depressions 2a may be specular to the frontal plane 1c and, therefore, the sagittal section is symmetrical in relation to the frontal plane 1c.

The base body 2 may have the minimum sagittal height at the bottom of the at least one depression 2a and, preferably, the maximum sagittal height at the apex of the at least one depression 2a.

In this document, the expression "sagittal height" identifies a length calculated perpendicular to the frontal plane 1c.

The minimum sagittal height of the base body 2 may be basically less than 100%, in detail less than 85%, and in more detail less than 65% of the maximum sagittal height of the same base body 2.

The profile of the depression 2a may be elliptical with a suitable eccentricity of less than 1, in detail less than 0.95, and in more detail basically ranging between 0.95 and 0.75, and preferably between 0.9 and 0.8.

The base body 2 has a frontal section (i.e. defined by the intersection of the frontal plane 1c with the base body 2) tapered with a minimum section close to the first end. In detail, the frontal section comprises a first proximal sector at the first end of a minimum and suitably constant frontal height; a second proximal sector at the second end of a maximum and suitably constant frontal height; and a central sector joining the first and second sectors and, thus, defining at least one slope **2b** between the first and second sectors.

In this document, the expression "frontal height" identifies a length calculated perpendicular to the sagittal plane 1b.

The central sector may have an increasing monotonic frontal height.

The at least one slope 2b is formed at the depressions 2a.

The radial section of the base body 2 made at the bottom of the depressions 2a has a frontal height and sagittal height basically equal to each other. Said radial section can preferably be circle-shaped.

The start of the slope 2b, i.e. the central sector, is at a shorter axial distance from the first end than the bottom of the depressions 2a.

In this document, the terms "radial" and "axial" identify a direction/plane respectively perpendicular and parallel to the longitudinal axis 1a.

The maximum frontal height and maximum sagittal height may be almost equal. The minimum frontal height may be less than the minimum sagittal height.

The central sector has two slopes 2b on opposite sides to the sagittal plane 1b and suitably almost specular to each other in relation to the sagittal plane 1b. Consequently, the central sector and, thus, the base body 2 may be specular to the sagittal plane 1b.

The base body 2 may be hollow.

The distractor 1 comprises a unit **3** for locking the distractor 1 at the spinous processes and, to be precise, between the spinous processes.

The unit 3 (also called herein anchoring unit or locking unit) is attached to the base body 2 at the first end and can be moved in relation to the body 2 so as to expand radially (in particular perpendicularly to the frontal plane 1c) locking the distractor 1 between the spinous processes.

The unit 3 is designed to define at least one expanded position wherein it has a maximum radial expansion; and at least one contracted position wherein it has a minimum radial expansion.

The unit 3 defines the head of the distractor 1 entering between the spinous processes.

The unit 3 may comprise at least one wing hinged to the base body 2 at the first end defining the rotation axis, which is, suitably, almost parallel to the frontal plane 1c. In detail, it comprises a first wing **3a** hinged to the base body 2 and a second wing **3b** hinged to the base body 2 on the opposite side to the first wing 3a in relation to the frontal plane 1c.

The wings 3a and 3b may have rotation axes that are basically parallel to each other. The wings 3a and 3b may rotate simultaneously and in the opposite direction during a change in the position of the unit 3.

They may have a tapered axial profile with a minimum section distal to the base body 2 so that, in the contracted position, the locking unit 3 is axially tapered and, in particular, ogive.

The wings 3a and 3b may have a U-shaped radial section with an opening facing the longitudinal axis 1a.

The distractor 1 may comprise an additional unit **4** for locking the distractor 1 at the spinous processes and, to be precise, between the spinous processes.

The additional unit 4 is attached to the base body 2 at the second end and can be moved in relation to the body 2 so as to expand radially (in particular perpendicularly to the frontal plane 1c) locking the distractor 1 between the spinous processes. The additional unit 4 is designed to define an additional expanded position wherein it has maximum radial expansion; and an additional contracted position wherein it has minimum radial expansion.

The additional unit 4 may comprise at least one additional wing hinged to the base body 2 at the second end with the rotation axis suitably almost parallel to the frontal plane 1c. In detail, it comprises a first additional wing **4a** hinged to the base body 2 and a second additional wing **4b** hinged to the base body 2 on the opposite side to the first additional wing 4a in relation to the frontal plane 1c.

The additional wings 4a and 4b have rotation axes basically parallel to each other and, in detail, to the wings 3a and 3b.

The additional wings 4a and 4b rotate simultaneously and in the opposite direction during a change in the position of the additional unit 4.

The distractor 1 comprises an actuator **5** designed to control the passage of the distractor 1 between an anchoring configuration and an un-anchoring configuration. In the anchoring configuration, the unit 3 is in the expanded position locking the distractor 1 in the correct position; and, suitably, the additional unit 4 is in the additional expanded position locking the distractor 1 on the opposite side of the unit 3 in relation to the depression 2a.

In the un-anchoring configuration, the unit 3 is in the contracted position and not locking the distractor 1 in the correct position; and, suitably, the additional unit 4 is in the additional contracted position.

The actuator 5 is designed to rotate, specifically exclusively, about the longitudinal axis 1a in relation to the base body 2 by controlling a change in the configuration of the distractor 1.

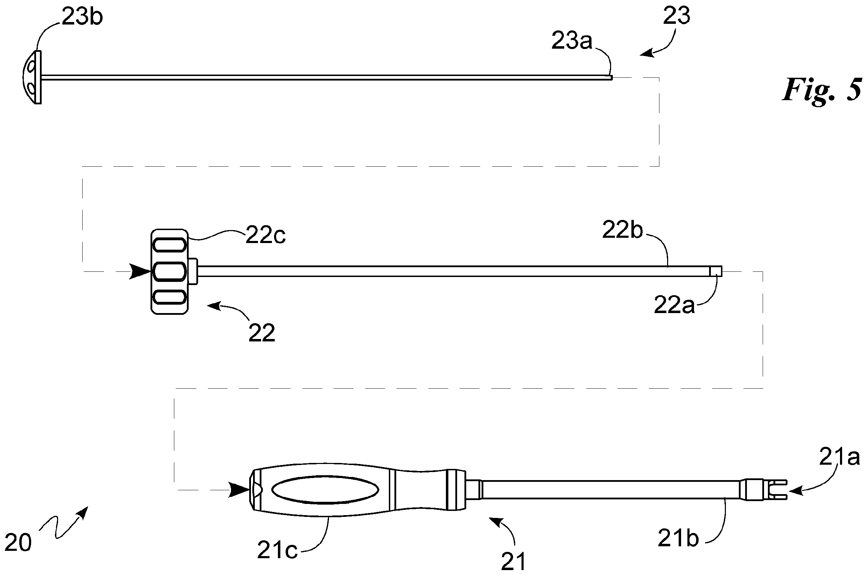

The actuator 5, shown in Fig. 5, may comprise a control thread **5a** of the unit 3 and, suitably, an additional control thread **5b** of the additional unit 4.

The additional thread 5b may be opposite the thread 5a.

To this end, the unit 3 may comprise, for each wing 3a and/or 3b, at least one thread 5a engagement tooth **3c** that, when pushed by the actuator 5, controls the rotation of the wings 3a and/or 3b.

The additional unit 4 may comprise, for each additional wing 5a and/or 5b, at least one additional thread 5b engagement tooth **4c** that, when pushed by the actuator 5, controls the rotation of the additional tooth 4c by controlling the rotation of the additional wings 4a and/or 4b.

The actuator 5 is externally controlled and the base body 2 comprises an opening **2c** for accessing the actuator 5 at the second end.

The opening 2c is always in view, i.e. accessible from the outside, regardless of the configuration of the distractor 1. The additional unit 4 in the contracted position is, therefore, folded over the base body 2 leaving the opening 2c in view.

The actuator 5 is at least partially housed in the base body 2.

The distractor 1 can be part of an interspinous vertebral distraction device **10**.

The distraction device 10 comprises the distractor 1, previously described, and a control **20** for the distractor 1.

The control 20, as presented in Figs. 4 and 5, defines a main extension axis **20a** that is preferably barycentric.

It is designed to be engaged with the distractor 1 at the opening 2c suitably arranging the axes 1a and 20a basically parallel to each other and, more specifically, so that they basically coincide.

The control 20 may comprise a first tool **21** for controlling the rotation of the distractor 1.

The first tool 21 is designed to define a first rotational constraint with, suitably exclusively, the base body 2 i.e. a constraint that prevents their mutual rotation about the axes 1a and/or 20a.

It comprises first means **21a** designed to be engaged with the body 2, suitably at the opening 2c, defining said first rotational constraint.

The first means 21a may comprise at least one tip and the base body 2 may comprise at least one seat **2d** for inserting said tip. In particular, they comprise two tips located on opposite sides of the axis 1a and the base body 2 comprises a seat 2d for each tip.

The first tool 21 comprises a hollow channel **21b** and, thus, defines a channel designed to be placed in communication with the opening 2c when the first tool 21 is attached to the base body 2.

The channel 21a is parallel to the main extension axis 20a.

The first means 21b protrude from the channel 21b along the main extension axis 20a.

The first tool 21 may comprise a grip **21c** connected to the channel 21b on the opposite side to the means 21a.

The control 20 may comprise a second tool **22** for controlling the change in configuration of the distractor 1.

The second tool 22 is designed to define a second rotational constraint with, suitably exclusively, the actuator 5 i.e. a constraint that prevents their mutual rotation about the axes 1a and/or 20a.

It comprises second means **22a** designed to be engaged with the actuator 5, suitably at the opening 2c, defining said second rotational constraint

The second constraint is basically an interlocking mechanism. For example, the actuator 5 may comprise a polygonal (e.g. hexagonal) end that is in view of the opening 2c and the second means 22a may define a counter-shaped engagement seat at said end.

The second tool 22 may rotate in relation to the base body 2 and the first tool 21 about the extension axis 20a.

It can be inserted into the channel 21a.

The second tool 22 may comprise an additional hollow channel **22b** and define an additional channel designed to be placed in communication with the opening 2c when the second tool 22 is attached to the actuator 5.

The additional channel 22a is parallel to the main extension axis 20a.

The second tool 22 may comprise a head **22c** for controlling the second tool 22.

The head 22c is designed to be placed outside the first tool 21 when the second tool 22 is in the channel 21b.

The control **20** may comprise a third tool **23** axially constraining at least one of the tools 21 and 22 to the distractor 1.

The third tool 23 is designed to be engaged with the actuator 5 by defining said axial constraint and, thus, preventing axial motion of the first tool 21 and/or of the second tool 22 in relation to the distractor 1.

It may comprise third means **23a** defining said axial constraint.

The third tool 23 may comprise a gripping element **23b** for controlling the third tool 23.

The gripping element 23b is designed to be placed outside the first tool 21 and the second tool 22 when the third tool 23 is in the additional channel 22b.

The third means 23a may define a threaded coupling with the actuator 5.

The third tool 23 can be at least partially arranged in the additional channel.

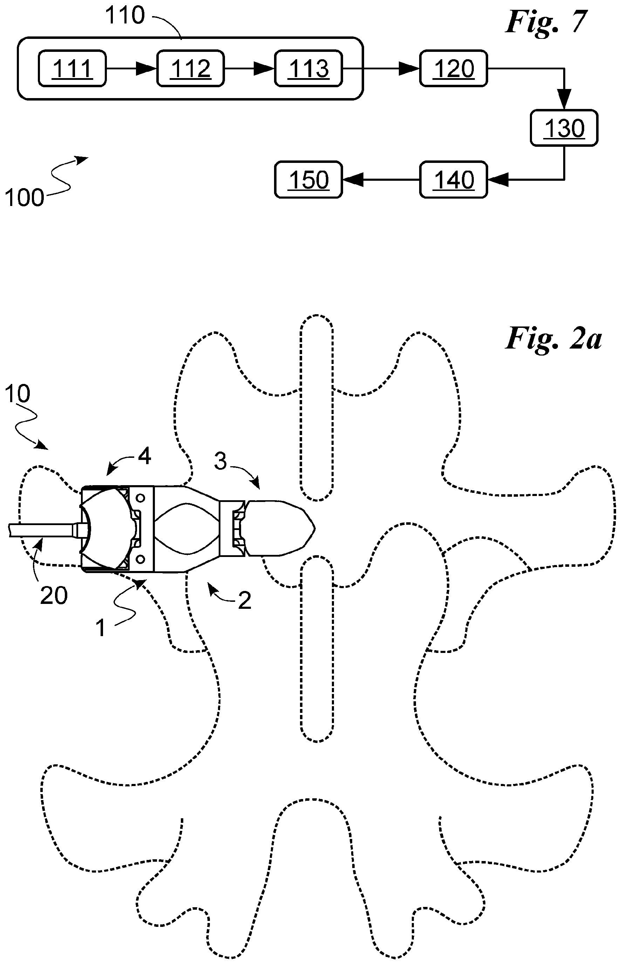

The operation of the distractor 1 and of the distraction device 10, described above in structural terms, define an innovative interspinous vertebral distraction procedure **100** that is schematised in Fig. 7.

The procedure 100 comprises the distractor 1 and, in particular, the distraction device 10.

The procedure 100 may comprise a preparation step **110** in which the control 20 is attached to the distractor 1.

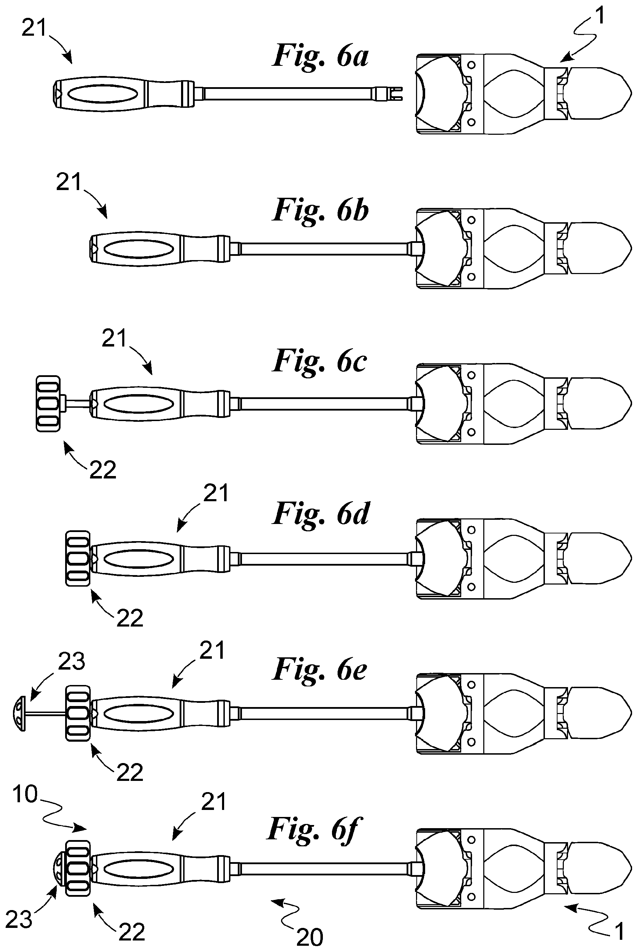

The preparation step 110 may comprise a first constraint sub-step **111** (Figs. 6a-6b) wherein the first tool 21 is engaged to the base body 2 defining said first rotational constraint. In detail, the first constraint is implemented thanks to the first means 21a by inserting the at least one tip in the at least one seat 2b.

The preparation step 110 may comprise a second constraint sub-step **112** (Figs. 6c-6d) wherein the second tool 22 is engaged with the actuator 5 defining said second rotational constraint. In detail, the first constraint is implemented by coupling the second means 22a to the actuator 5 by interference.

In the sub-step 112, the second tool 22 is inserted into the channel 21b.

The preparation step 110 may comprise a third constraint sub-step **113** (Figs. 6d-6e) wherein the third tool 23 is designed to be engaged to the actuator 5 defining said axial constraint.

At the end of the preparation step 110, the distractor 1 is in the un-anchoring configuration.

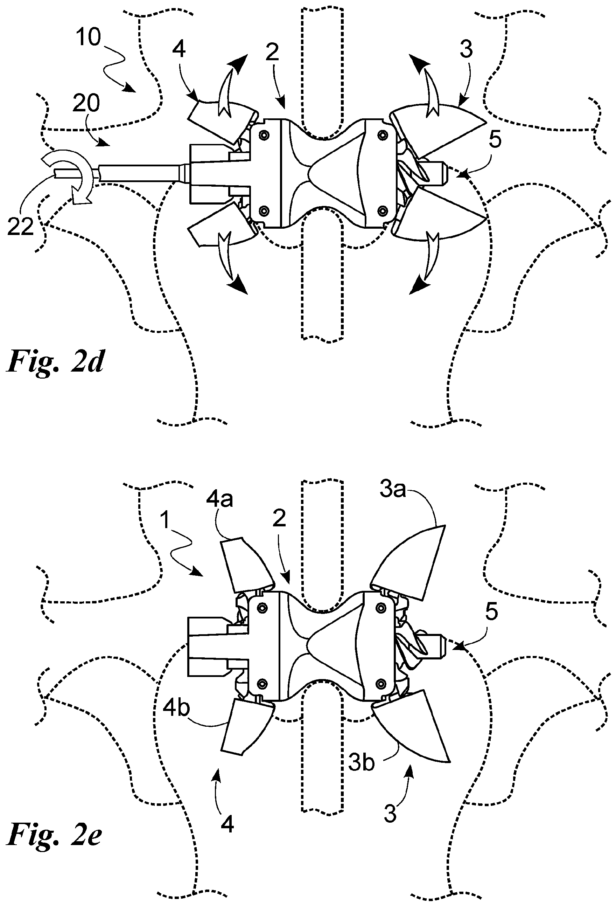

The procedure 100 may comprise a positioning step **120** (Figs. 2a-2b) wherein the distractor 1, suitably controlled by the control 20 and, in particular, by the first tool 21, is brought near the spinous processes.

In this step 120, the distractor 1 is placed with the frontal plane 1c basically perpendicular to the patient's frontal plane and then moved along the longitudinal axis 1a bringing the locking unit 3 basically to the two spinous processes. The distractor 1 then moves axially forward so that the unit 3, in the contracted position, penetrates between the spinous processes and, thanks to its tapered profile, is arranged between them.

The step 120 ends when the at least one slope 2b is proximal to the spinous processes.

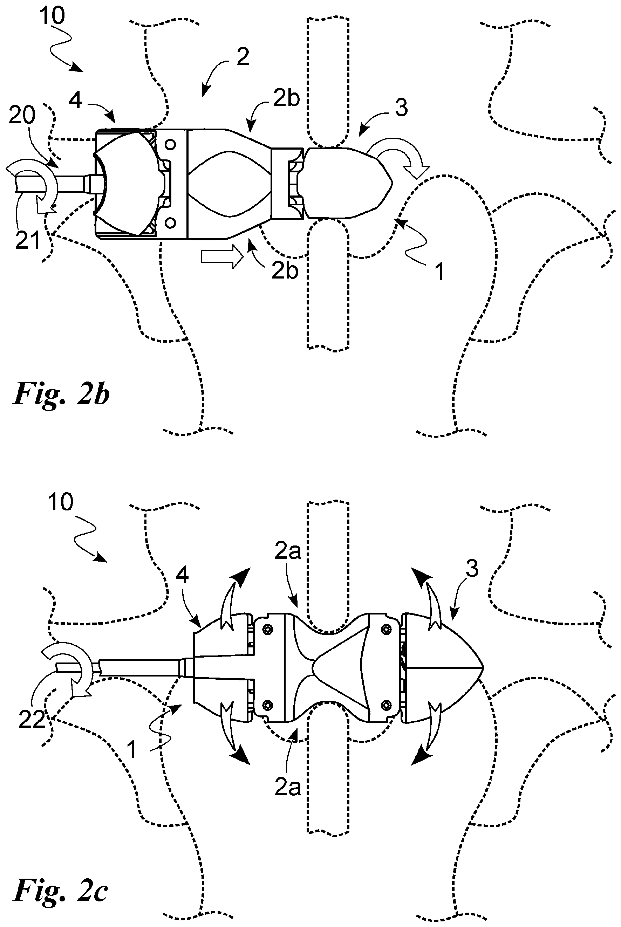

The procedure 100 may comprise an insertion step **130** (Figs. 2b-2c) wherein the base body 2 and, in detail, the entire distractor 1 rotate about the longitudinal axis 1a arranging the frontal plane 1c basically parallel to the patient's frontal plane and, thus, the two spinous processes in the depressions 2a.

In this step 130, the first tool 21 rotates the distractor 1 and, to be precise, axially moves the distractor 1 and, preferably simultaneously, rotates it about the longitudinal axis 1a.

The axial translation causes the spinous processes to slide along the slopes 2b so that they gradually adapt to the profile of the sagittal section and, thus, to the at least one depression 2a.

The rotation enables the depressions 2a to appear and, thus, to house the spinous processes inside, suitably after they have travelled at least part of the slopes 2b.

During the insertion step 130, the distractor 1 is rotated by an angle basically ranging between 60° and 120°, in detail between 75° and 105°, and preferably around 90°. The procedure 100 may comprise an expansion step **140** (Figs. 2c-2d) wherein the distractor 1 switches from the anchoring configuration to the un-anchoring configuration.

In the expansion step 140, the actuator 5, controlled by the second tool 22, causes the unit 3 to pass into the expanded position so as to constrain the distractor 1 between the spinous processes.

At the same time, the actuator 5 suitably controls the passage of the additional unit 4 into the additional expanded position so as to constrain the distractor 1 between the spinous processes on the opposite side to the unit 3 in relation to the depressions 2a.

It should be noted that the variation in the configuration of the distractor 1 is controlled by rotating the second tool 22 in relation to the first tool 21 that, suitably, remains substantially still.

The procedure 100 may comprise a removal step **150** (Fig. 2e) for moving the control 20 away from the distractor 1.

In this step 150, the third tool 23 is disengaged from the actuator 5; then the second tool 23 and then the first tool 21 are moved away from the distractor 1.

The distractor 1 according to the invention the vertebral distraction device 10 and, thus, the interspinous vertebral distraction procedure 100 (not claimed) achieve important advantages.

In fact, thanks to the particular conformation of the distractor 1 and/or the practicality of the distractor control defined by control 20, they are particularly simple and quick to use and, in particular, enable a precise, firm, and rapid positioning of the distractor 1 between the spinous processes.

An important advantage is, therefore, the high stability of the distractor 1 once implanted. In fact, thanks to the depressions 2a, for example, the distractor 1 has a precise housing for each spinous process.

These advantages are enhanced by the presence of slopes 2b that, by gradually spacing the spinous processes apart, make it possible to carry out both a rapid and precise positioning of the distractor 1 and a less invasive procedure for the patient. Another important advantage is the particular control 20 that makes it possible to have a precise and practical control of the distractor 1 and, in particular, of the wings 3a and 3b and, suitably, of the additional wings 4a and 4b.

A significant advantage is that the distractor 1 (and, thus, the vertebral distraction device 10 and procedure 100) have low costs.

## Claims

1. An interspinous vertebral distractor (1) designed to be placed between two spinous processes and defining a longitudinal axis (1a), a sagittal plane (1b), and a frontal plane (1c); said distractor (1) comprising
- a base body (2) designed to be placed between said two spinous processes and defining, in relation to said longitudinal axis (1a), a first end and a second end;
- an anchoring unit (3) firmly attached to said base body (2) at said first end and designed to expand perpendicularly to said frontal plane (1c);
- an actuator (5) designed to control the passage of said unit (3) between an expanded position wherein said unit (3) constrains said distractor (1) between said two spinous processes and a contracted position wherein said unit (3) does not constrain said distractor (1) between said two spinous processes; and wherein
- said base body (2) has a sagittal section with two opposing convexities so as to define a profile with two depressions (2a) placed on the opposite side of said frontal plane (1c) each of which is designed to accommodate one of said two spinous processes,
**characterised in that**
- said base body (2) has a tapered frontal section with the minimum section at the first end;
- wherein the frontal section of said base body (2) comprises a first sector, a second sector, and a central sector;
- wherein said first sector is at said first end and has the minimum height, wherein said second sector is at said second end and has the maximum height; wherein said central sector defines two slopes (2b) joining said first sector and said second sector placed on the opposite side to said sagittal plane (1b).

2. The distractor (1) according to the previous claim, wherein said depressions (2b) are basically formed on at least one slope (2b).

3. The distractor (1) according to the previous claim, wherein the beginning of said slope (2b) has a shorter axial distance from said first end than the bottom of said depressions (2a).

4. The distractor (1) according to at least one claim from 2-3, wherein the radial section of said base body (2) made at said bottom of said depressions (2a) has a frontal height and sagittal height basically equal to each other.

5. The distractor (1) according to at least one previous claim, wherein the sagittal height of said base body (2) at the bottoms of said depressions (2a) is basically less than 85% of the sagittal height of said base body (2) at the apexes of said depressions (2a).

## Patentansprüche

1. Interspinöser Wirbelspreizer (1), der dazu ausgelegt ist, zwischen zwei Dornfortsätzen platziert zu werden und eine Längsachse (1a), eine Sagittalebene (1b) und eine Frontalebene (1c) definiert; wobei der Spreizer (1) umfasst:
- einen Grundkörper (2), der ausgelegt ist, zwischen den beiden Dornfortsätzen platziert zu werden und in Bezug auf die Längsachse (1a) ein erstes Ende und ein zweites Ende zu definieren;
- eine Verankerungseinheit (3), die fest mit dem Grundkörper (2) an dem ersten Ende befestigt ist und dazu ausgelegt ist, sich senkrecht zur Frontalebene (1c) auszubreiten;
- einen Aktuator (5), der dazu ausgelegt ist, den Durchgang der Einheit (3) zwischen einer ausgefahrenen Position, in der die Einheit (3) den Spreizer (1) zwischen den beiden Dornfortsätzen blockiert, und einer eingefahrenen Position, in der die Einheit (3) den Spreizer (1) zwischen den beiden Dornfortsätzen nicht blockiert, zu steuern; wobei
- der Grundkörper (2) einen Sagittalschnitt mit zwei gegenüberliegenden Konvexitäten aufweist, um ein Profil mit zwei Vertiefungen (2a) zu definieren, die sich auf der gegenüberliegenden Seite der Frontalebene (c) befinden, von denen jede dazu ausgelegt ist, einen der beiden Dornfortsätze aufzunehmen,
**dadurch gekennzeichnet, dass**
- der Grundkörper (2) einen verjüngten Frontalschnitt mit dem Minimalschnitt an dem ersten Ende aufweist;
- wobei der Frontalschnitt des Grundkörpers (2) einen ersten Bereich, einen zweiten Bereich und einen Zentralbereich umfasst;
- wobei der erste Bereich an dem ersten Ende liegt und die Minimalhöhe aufweist, wobei der zweite Bereich an dem zweiten Ende liegt und die Maximalhöhe aufweist; wobei der Zentralbereich zwei Neigungen (2b) definiert, die den ersten Bereich und den zweiten Bereich auf der gegenüberliegenden Seite der Sagittalebene (1b) verbinden.

2. Spreizer (1) gemäß dem vorhergehenden Anspruch, wobei die Vertiefungen (2b) im Wesentlichen auf mindestens einer Neigung (2b) ausgebildet sind.

3. Spreizer (1) gemäß dem vorhergehenden Anspruch, wobei der Neigungsanfang (2b) einen kürzeren axialen Abstand von dem ersten Ende als der Boden der Vertiefungen (2a) aufweist.

4. Spreizer (1) gemäß mindestens einem der Ansprüche 2-3, wobei der Radialschnitt des Grundkörpers (2) an dem Boden der Vertiefungen (2a) eine Frontalhöhe und eine Sagittalhöhe aufweist, die im Wesentlichen gleich miteinander sind.

5. Spreizer (1) gemäß mindestens einem der vorhergehenden Ansprüche, wobei die Sagittalhöhe des Grundkörpers (2) am Boden der Vertiefungen (2a) im Wesentlichen weniger als 85 % der Sagittalhöhe des Grundkörpers (2) an den Spitzen der Vertiefungen (2a) beträgt.

## Revendications

1. Distracteur vertébral interépineux (1) conçu pour être placé entre deux apophyses épineuses et définissant un axe longitudinal (1a), un plan sagittal (1b), et un plan frontal (1c); ledit distracteur (1) comprenant
- un corps de base (2) conçu pour être placé entre lesdites deux apophyses épineuses et définissant, par rapport audit axe longitudinal (1a), une première extrémité et une seconde extrémité;
- une unité d'ancrage (3) fermement attachée audit corps de base (2) à ladite première extrémité et conçue pour s'étendre perpendiculairement audit plan frontal (1 c);
- un actionneur (5) conçu pour contrôler le passage de ladite unité (3) entre une position étendue où ladite unité (3) contraint ledit distracteur (1) entre lesdites deux apophyses épineuses et une position contractée où ladite unité (3) ne contraint pas ledit distracteur (1) entre lesdites deux apophyses épineuses; et dans lequel
- ledit corps de base (2) a une section sagittale avec deux convexités opposées de manière à définir un profil avec deux dépressions (2a) placées du côté opposé dudit plan frontal (1c), chacune conçue pour accueillir l'une desdites deux apophyses épineuses,
**caractérisé en ce que**
- ledit corps de base (2) a une section frontale effilée avec la section minimale à la première extrémité;
- dans lequel la section frontale dudit corps de base (2) comprend un premier secteur, un second secteur et un secteur central;
- dans lequel ledit premier secteur est à ladite première extrémité et a la hauteur minimale, dans lequel ledit second secteur est à ladite seconde extrémité et a la hauteur maximale; dans lequel ledit secteur central définit deux pentes (2b) joignant ledit premier secteur et ledit second secteur placées du côté opposé audit plan sagittal (1b).

2. Le distracteur (1) selon la revendication précédente, dans lequel lesdites dépressions (2b) sont essentiellement formées sur au moins une pente (2b).

3. Le distracteur (1) selon la revendication précédente, dans lequel le début de ladite pente (2b) a une distance axiale plus courte depuis ladite première extrémité depuis le fond desdites dépressions (2a).

4. Le distracteur (1) selon au moins une des revendications 2-3, dans lequel la section radiale dudit corps de base (2) réalisée audit fond desdites dépressions (2a) a une hauteur frontale et une hauteur sagittale essentiellement égales l'une à l'autre.

5. Le distracteur (1) selon au moins une revendication précédente, dans lequel la hauteur sagittale dudit corps de base (2) au fond desdites dépressions (2a) est essentiellement inférieure à 85 % de la hauteur sagittale dudit corps de base (2) aux sommets desdites dépressions (2a).
